Europäisches Patentamt

European Patent Office

Office européen des brevets

(19)

(11) Publication number: **0 345 543 B1**

(12) **EUROPEAN PATENT SPECIFICATION**

(45) Date of publication of patent specification: **12.10.94**  (51) Int. Cl.⁵: **A61K 39/395**

(21) Application number: **89109372.6**

(22) Date of filing: **24.05.89**

(54) **Therapeutic IgM concentrates.**

(30) Priority: **06.06.88 US 203377**

(43) Date of publication of application:
**13.12.89 Bulletin 89/50**

(45) Publication of the grant of the patent:
**12.10.94 Bulletin 94/41**

(84) Designated Contracting States:
**AT BE CH DE ES FR GB GR IT LI LU NL SE**

(56) References cited:
**EP-A- 0 162 462**
**US-A- 3 597 409**
**US-A- 3 808 189**

**DICTIONNAIRE VIDAL, 63th ed., 1987; p. 777&NUM;**

**ROTE LISTE, 1986, Editio Cantor; no. 74019&NUM;**

**ENZYME MICROB. TECHNOL., vol. 9, June 1987, Butterworth Publishers; F. STEINDL et al., pp. 361-364&NUM;**

**JOURNAL OF CHROMATOGRAPHY, vol. 358, 1986, Elsevier Science Publishers B.V., Amsterdam (NL); P. CLEZARDIN, pp.**

**209-218&NUM;**

(73) Proprietor: **MILES INC.**
**1127 Myrtle Street**
**Elkhart Indiana 46514 (US)**

(72) Inventor: **Collins, Michael S.**
**2816 Wright Avenue**
**Pinole, CA 94564 (US)**
Inventor: **Opitz, Hans Georg**
**140 Clover Hill Ct.**
**Danville, CA 94526 (US)**
Inventor: **Lundblad, John L.**
**1390 Club View Ct.**
**El Cerrito, CA 94530 (US)**
Inventor: **Seng, Richard L.**
**P.O. Box 2038**
**Guerneville, CA 95446 (US)**

(74) Representative: **Dänner, Klaus, Dr. et al**
**Bayer AG**
**Konzernverwaltung RP**
**Patente Konzern**
**D-51368 Leverkusen (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This disclosure is concerned generally with antibody preparations having a prophylactic or therapeutic effect. Specifically, the disclosure is concerned with such preparations that have been enriched in antibodies of the IgM type, the preparations are low in homologous isoagglutinins, and they are found to be useful in treatment of infections of gram negative bacteria.

Antibodies may be classified according to a well known typing system (i.e. IgG, IgM, IgA, IgD, IgE) and, in the case of IgG, according to sub-types (i.e. $IgG_1$, $IgG_2$, $IgG_3$, and $IgG_4$).

Commercially available antibody preparations (known as immune serum globulin or ISG) commonly consist mainly of antibodies of the IgG type with the distribution of IgG sub-types approximating that found in human plasma. Typically, the amount of IgM in such preparations, if present at all, is relatively small.

IgM is a well known 19S immunoglobulin which comprises about 7% of the immunoglobulins found in man. IgM antibodies are said to have an antibody valence of at least five and they are the earliest antibodies generated in an immune response. Although IgM antibodies tend to be very effective, especially in combating bacterial infections, they have a relatively short in vivo half life of about five days. Further, IgM antibodies tend to aggregate and are relatively difficult to stabilize, especially in purified form.

To date, the only known commercial intravenous (IV) product having significant amounts of IgM antibody is a product known as Pentaglobin™, available from Biotest, GmbH, of West Germany. The use of that product appears to be described in an article by K.D. Tympner, et al, "Intravenose IgM-Applikation", Mschr. Kinderheilk. 123,400-401 (1975). That product comprises, on a percent by weight total protein basis, about 76% IgG, about 12% IgA and about 12% IgM.

It has been thought that the use of larger amounts of IgM in an ISG product could lead to adverse reactions. For example, it is known that IgM is many times more potent than IgG in activating complement in an immune reaction. This is because one molecule of IgM bound to an antigen will activate complement whereas two or more molecules of IgG must be bound to an antigen in close association to each other to activate complement.

It appears that the very production methods used in preparing the existing product (involving the use of $\beta$-propriolactone to make the product IV administrable, as taught, for example, in U.S. Patent 4,318,902 to W. Stephan) may limit the amount of IgM available due to degradation reactions. Hence, for whatever reason, even though IgM is recognized as very effective, it has not appeared in any commercially available intravenously useful ISG product at an amount greater than about 12% by weight total protein. Although 20% by weight IgM products have been available in the past (Gamma-M-Konzentrat, Behringwerke AG, Marburg, Germany and "Immunoglobulines IgGAM", Bio-Transfusion, Lille, France), they have been made for and limited to intramuscular (not IV) applications.

Various purification schemes have been suggested for plasma-derived IgM and, more recently, monoclonal-derived IgM. In the case of plasma-derived IgM, it has been known since the 1940's that alcohol fractionation techniques could be used to obtain a relatively concentrated IgM from what is known as Cohn Fraction III. See also, for example, US-A-3 597 409, US-A-3 808 189, and the above-cited U.S. Patent 4,318,902 (and the cited references) to W. Stephan and concerned with the use of beta-propriolactone to make a concentrated (12%) IgM suitable for intravenous (IV) administration. In addition, see EPO application 0 038 667 of Mitra et al (IgM acylation). Other IgM purification or preparation techniques are disclosed by U. Sugg et al, Vox Sang. 36:25 - 28 (1979); M. Steinbuch et al, Preparative Biochemistry 3(4), 363 - 373 (1973) and A. Wichman et al, Biochem. Biophys. Acta 490:363 - 69 (1977). See also, U.S. Patent No. 4,272,521 to Zuffi concerned with the purification of immune serum globulins in general by using ion exchange resins at an alkaline pH. For a variety of technical reasons, plasma derived IgM has been relatively difficult to purify and the highest known purity to date (used in analytical purposes) is about 90% IgM, by weight.

Since the publication by Köhler and Milstein, "Continuous Cultures of Fused Cells Secreting Antibody of Predefined Specificity", Nature 256:495 - 497 (1975), the production of monoclonal antibodies has become well known. Monoclonal antibodies of a given specificity are now routinely made using somatic cell hybrids (see, for example, U.S. Patent No. 4,172,124 to H. Koprowski et al), using EBV transformed cells (see, for example, U.S. Patent No. 4,446,465 to M. Lostrom), a combination of the two, or by the electrofusion of cells. Monoclonals of both the IgG and IgM classes have been made, purified and characterized. Such IgM preparations are described by D. Nau, Biochromatography, 1, No. 2, pp. 83 - 84 (95% pure IgM from tissue culture); M. Fishner, U.S. Patent No. 4,604,235 (90% pure IgM from mouse ascites fluid and which was characterized as "essentially pure antibody"); J. R. Wands et al, W 082/01072 (high affinity IgM monoclonal antibodies for diagnostics; S. Burchiel, et al, J. Immuno. Meth., 69, p. 33, 1984 (IgG purified from mouse ascites fluid); J. Deschamps et al, Anal. Biochem. 147, p. 451, 1985 (IgG from mouse and human IgG and IgM) Steindl et al, Enzym. Microb. Technol., Vol. 9, 1987, p. 361. Notwithstanding the above recent work, however, to date we are unaware of any concentrated IgM product, as defined herein, that is both stable and highly effective in prophylaxis and treatment of infections of gram negative bacteria.

Surprisingly, we have found that therapeutically effective and highly concentrated IgM preparations can be prepared from plasma sources. Those preparations are stable and considerably more effective than existing ISG preparations and even the only enriched, intravenous IgM product, described above. Details of our IgM concentrate, its manufacture and use are described herein.

SUMMARY OF THE INVENTION

Our therapeutic IgM concentrate comprises a substantially pure antibody composition comprising an IgM concentrate having at least about 33% by weight of antibodies of the IgM type and an isoagglutinin titer of less than about 1:4. The preparation is stable and may be prepared in a lyophilized or freeze-dried form. It is polyclonal and obtained from a plasma source. The IgM concentrate is effective against a wide range of pathogens, especially gram negative enteric bacteria. Upon aqueous reconstitution, the lyophilized preparation results in a pharmaceutically acceptable IV solution having a pH ranging from about 4.0 to 8.0, preferably 4.0 to 7.0. In practical use, the concentrate may contain heterologous isoaggutinins as long as the homologous isoagglutinin titer is less than about 1:4. In liquid form suitable for IV administration, the IgM concentration is at least 5 mg per ml.

The IgM concentrate of this disclosure may be prepared as follows from human plasma using any of the examples shown below.

SPECIFIC EMBODIMENTS

An important aspect of this disclosure is based on our discovery that an IgM concentrate, substantially free of isoagglutinins as those terms are defined herein, is surprisingly efficacious against gram negative bacteria.

As used herein, IgM concentrate, or its equivalent, means an antibody preparation comprising at least about 33% by total weight of biologically active antibodies of the IgM type.

Substantially free of isoagglutinins means having an homologous isoagglutinin titer of less than 1:4. Ideally, the titer of all isoagglutinins is less than 1:4. As a practical matter, the IgM concentrate of this disclosure must be at least substantially free of homologous isoagglutinins (i.e. isoagglutinins of the type that can complex in a patient having a blood type against which the isoagglutinins are specific).

Pharmaceutically acceptable means capable of being administered to a mammal (such as a human) intravenously without deleterious effect. Numerous aqueous vehicles for safely intravenously administering antibodies to a patient are well known. See, for example, U.S. Patent 4,396,608 to Tenold and U.S. Patent 4,186,192 to Lundblad et al.

Protective effect against such organisms means that the prophylactic administration of the IgM concentrate of this disclosure significantly lowers mortality in infected animals (as shown in the protective effect examples below).

In the examples below, concentrated IgM products of this disclosure were compared with a commercially available Intravenous Immune Globulin (IVIG) known as Gamimune®-N (Cutter Biological, Miles Inc.), and an "enriched IgM" (or 12% by weight) product known as Pentaglobin® (Biotest, GmbH).

The respective amounts of each of the antibody types of each of the products used below are summarized in Table 1.

Table 1

| | Pentaglobin®** | High* (97.2%) IgM Conc. | Med.* (60.4%) IgM Conc. | Gamimune®-N IVIG, pH 4.25 |
|---|---|---|---|---|
| Protein | 5.0 grams | 5.0 grams | 5.0 grams | 5.0 grams |
| IgM | 0.6 grams | 4.86 grams | 3.02 grams | trace < 0.1g |
| IgA | 0.6 grams | 0.07 grams | 1.22 grams | trace < 0.1g |
| IgG | 3.8 grams | 0.07 grams | 0.76 grams | 4.98 grams |
| Ratio, IgM:IgG | 0.16:1 | 70:1 | 5:1 | N.A. |
| IgM(wt %) | 12 | 97.2 | 60.4 | -- |

* wt. % total protein
** Having saline anti-A antibody isoagglutinin titer of 1:32 and anti-B isoagglutinin titer of 1:8

PREPARATION OF IGM CONCENTRATES

The plasma derived IgM of the examples below were derived from human plasma and initially found to contain antibodies isoagglutinins) which react with blood group specific antigens. It is known that such antibodies may cause severe side effects (i.e. complement activation or cell lysis if incubated with the appropriate erythrocytes). This is especially a problem in the presence of highly concentrated IgM since, as noted above, IgM is many times more potent than IgG in activating complement in an antibody-mediated immune response. To prevent this, any homologous isoagglutinins (and preferably all isoagglutinins) must be removed or reduced by further purification steps (e.g. using specific adsorbants such as Synsorb® A or Synsorb® B). Alternatively, the IgM concentrate may be prepared from pooled donations of blood of a common blood type (e.g. type A) and then identified for subsequent administration only to a patient of similar blood type. Also, one can simply contact the IgM concentrate with fixed RBCs or RBC stroma with which the undesired isoagglutinins will complex. Other methods of removing isoagglutinins or reducing the titer of a specific isoagglutinin to less than 1:4 may be possible.

Example 1

Fraction III was obtained from normal human plasma by means of the Cohn/Oncley cold ethanol method. Alternatively, precipitate B is equally acceptable and is obtained from normal human plasma by the method of Kistler and Nitschmann. One kg of Fraction III paste thus obtained was suspended at 20° C in 12.5 volumes of 0.05 M sodium acetate at pH 4.8. Caprylic acid was added to a concentration of 2.5% by vol/wt. After aging at 5° C for approximately 16 hours the insoluble impurities were removed by centrifugation and filtration. The filtrate was adjusted to 7.5% polyethylene glycol and the resulting IgM precipitate collected by centrifugation. The IgM paste was solubilized in phosphate buffer at pH 6.5 and virus inactivation was achieved by application of tri-n-butyl phosphate and Tween® 80. The virus-free solution was adjusted to 0.025 M sodium acetate, 0.2 M glycine., 0.05 M sodium chloride, pH 4.8. The IgM thus prepared has a globulin composition by weight of 56.8% IgM, 21.6% IgG, and 21.7% IgA and saline titer for anti A of 1:64 and a saline anti B titer of 1:64. These antibodies can be removed or reduced to a titer of less than 1:4 by the techniques described above.

Example 2

Fraction III was obtained from a blood type A specific pool of plasma (i.e. from donors having type A blood) and processed as in Example 1 through the caprylic acid precipitation, clarification and filtration steps. The filtrate was adjusted to 0.16 M sodium potassium phosphate at pH 6.5 and the IgM adsorbed on an anion exchanger such as Q Sepharose® equilibrated with the same pH 6.5 buffer. The adsorbed IgM was washed with equilibration buffer. IgM was eluted from the exchanger with 0.3 M sodium potassium phosphate at pH 6.5. Virus inactivation is achieved by application of tri-n-butyl phosphate and Tween® 80. The virus-free solution is adjusted to 0.025 M sodium acetate, 0.2 M glycine, 0.05 M sodium chloride, pH 4.8. The IgM thus prepared has a globulin composition by weight of 96% IgM, 1.3% IgG, 2.1% IgA, and a saline titer of less than 1 anti A.

Example 3

Fraction III paste was suspended in five volumes of water for injection at 5° C and then adjusted to pH 5.2 with the addition of 6.5 volumes buffer to achieve 0.03 M sodium acetate. The suspension was heated to 20° C and cooled slowly to 2° C, adjusted to pH 4.8, and impurities were precipitated by caprylic acid added to 2%. Precipitated impurities were removed by centrifugation and filtration. The thus prepared IgM filtrate was adsorbed on Q Sepharose® and further processed as in Example 2 with the exception of IgM elution from the anion exchanger which was accomplished with 0.3 M sodium acetate at pH 4.8. The IgM thus prepared has a globulin composition by weight of about 76.2% IgM, about 13.8% IgG and about 8.3% IgA.

In Example 4 below the IgM applied to animal studies was principally manufactured by Example 1 and to a lesser extent by Examples 2 and 3. The preferred method for manufacture is demonstrated in Example 2. In addition to removal of homologous isoagglutinms by type specific pooling of plasma, the use of synthetic type specific adsorbants would be preferred.

Initial Studies

Example 4

In all animal studies below, all aqueous IgM concentrate solutions were made by diluting or reconstituting IgM concentrates comprising at least 33% by weight of IgM antibodies (i.e. 33% of total antibodies were of the IgM type). The minimum IgM concentration was 50 mg/kg of animal treated.

Assessment of plasma-derived IgM binding to pathogenic bacteria by the indirect immunofluorescence assay (IFA).

Standard suspensions of 33 pathogenic bacteria were prepared. Bacteria were harvested in mid-log phase by washing cells off agar growth medium with saline. Cells were washed once with saline and resuspended in saline. Formalin (37% formaldehyde) was added to 1% vol/vol, and the cell suspensions were held at 4° C for 24 hours. Cells were washed once with sterile saline and resuspended in saline to an optical density (OD) of 0.20 at 660 nm. For the IFA, 100 $\mu$l of bacterial cell suspension was added to 100 $\mu$l of IgM solution containing 270 $\mu$g IgM/ml. The mixture was incubated for 30 minutes at 37° C. Cells were pelleted by centrifugation, washed once with saline and repelleted by centrifugation. To detect bound IgM, 50 $\mu$l of a 1:30 dilution of fluorescein-labeled goat anti-human IgM ($\mu$ chain specific-Hyclone Laboratories, Inc., Logan UT) was added to the bacterial cell pellet. An aliquot of the mixture was transferred to a microscope slide and fluorescence was assessed at 510-560 nm by microscopy.

Table 2

| Binding of plasma-derived IgM to pathogenic bacteria: Assessment of the reactivity of IgM by the IFA. | |
|---|---|
| Organisms | No. strains positive/total |
| Pseudomonas aeruginosa | 6/6 |
| P. cepacia | 3/3 |
| P. fluorescense | 1/1 |
| Salmonella typhimurium | 5/5 |
| Klebsiella pneumoniae | 5/5 |
| Enterobacter aerogenes | 4/4 |
| E. cloacace | 1/1 |
| Proteus mirabilis | 5/5 |
| Serratia marcescens | 5/5 |
| Haemophilus influenzae | 1/3 |
| Enterococcus (Group D Streptococcus) | 4/5 |
| Total | 30/33 90.9% |

Binding of immunoglobulin to the microbial cell surface is the initial step in antibody-mediated opsonization and subsequent engulfment and killing of the bacterium by a phagocyte. The results in Table 2 indicate that plasma-derived IgM bound to all <u>Pseudomonas</u> sp. and enteric bacilli examined. IgM binding to <u>H</u> <u>influenzae</u> and enterococci was variable.

Example 5

Comparative opsonic activity of plasma-derived IgM and IgG.

A phagocytic assay was employed to compare the opsonic activity of plasma-derived IgM with a commercially available plasma-derived IgG (Gamimune®-N, Miles Inc., Berkeley, CA). The phagocytic assay employed bacteria and human phagocytes (PMNs) suspended in tissue culture fluid. The bacteria: PMN ratio was 10:1. The complement source was 2% (vol/vol) guinea pig serum (GPS). Heated GPS was included in the assay to assess the effect of immunoglobulin alone or other heat stable, non-antibody opsonic serum factors. After 100 min incubation at 37° C, an aliquot of the assay mixture was added to 9 vol. of distilled water to lyse PMNs and surviving bacteria were enumerated by duplicate agar plate counts. Two strains of <u>Escherichia</u> <u>coli</u> K1 were examined in the assay.

Approximately 84% of the strains of E. coli associated with neonatal meningitis have the K1 capsular polysaccharide antigen. (Robbins, J.B., et al. 1974. Escherichia coli capsular polysaccharide associated with neonatal meningitis. N. Engl. J. Med. 290:1216-1220). The results presented in Table 3 indicate that plasma-derived IgG at 10.0 mg/ml had no opsonic activity against two K1 strains of this important human pathogen. In contrast plasma-derived IgM at a concentration 20-fold less than IgG (0.5 mg/ml promoted > 1 $\log_{10}$ reduction of the E. coli K1 inocula. The opsonic activity of IgM was complement-dependent for a significant ($\geq$ 80%) reduction of the inocula did not occur when heated GPS (complement-inactivated) was present in the assay. The heated GPS control also indicated that the IgM-mediated reduction in E. coli K1 inocula was not due to bacterial agglutination.

### Table 3

Plasma-derived IgM versus IgG Opsonic Activity: $Log_{10}$ Cell Reduction at 100 min.

| Bacterial strain | Immunoglobulin | Complement (2% GPS) | Immunoglobulin Concentration mg/ml | | | | |
|---|---|---|---|---|---|---|---|
| | | | 0 | 0.1(-)[a] | 0.5(0.4) | 2.7(2.0) | -(10.0) |
| E. coli 016:K1 | IgM | Active | 0.73 | 0.62 | 1.68[c] | 2.43[c] | ND |
| | IgM | Heated | 0.55 | 0.61 | 0.57 | 0.87 | ND |
| | IgG | Active | 0.73 | ND | 0.68 | 0.70 | 0.51 |
| | IgG | Heated | 0.55 | ND | 0.65 | 0.64 | 0.38 |
| E. coli 050:K1 | IgM | Active | 0.70 | 0.61 | 2.17[c] | 2.69[c] | ND |
| | IgM | Heated | 0.65 | 0.54 | 0.67 | 1.11 | ND |
| | IgG | Active | 0.70 | ND | 0.67 | 0.72 | 0.62 |
| | IgG | Heated | 0.65 | ND | 0.54 | 0.58 | 0.54 |

[a] IgM concentration (IgG concentration)

[b] ND is not done

[c] Significant killing; >80% reduction compared to immunoglobulin free guinea pig serum control.

Example 6

Plasma derived IgM activity versus IgG activity: Prophylaxis of infection in murine models

The antimicrobial activity of IgM was compared with IgG in a variety of animal models of infection. In all models of infection, bacterial inocula were prepared as mid-log phase cells following 4 to 5 hours growth at 37°C on appropriate agar media. The number of bacteria in the challenge inocula was determined by correlating absorption at 660 nm with predetermined plate counts. After mice were challenged with approximately 10 mean lethal doses of each strain, the exact number of cells in the inoculum was determined by duplicate plate counts on agar medium. Female Swiss-Webster mice of strain CrL:CFW-(SW)BR, weighing 22-26 g were obtained from Charles River Breeding Laboratories (Wilmington, MA). For each study mice were housed 10 per cage and given water and mouse chow ad lib. In all studies, immunoglobulin preparations were administered by the intraperitoneal route two to three hours before bacterial challenge.

Peritoneal infection. A 0.1 ml vol of cells suspended in sterile saline was injected by the intraperitoneal route.

Mucin-enhanced peritoneal infection. A 0.5 ml vol of cells suspended in 7% sterile hog gastric mucin (Sigma Chemical Co., St. Louis, Mo) was injected by the intraperitoneal route (Teng, N.H. et al. 1985. Protection against Gram-negative bacteremia and endotoxemia with human monoclonal IgM antibodies. Proc. Natl. Acad. Sci. 82:1790-1794).

Pneumonia. A 0.05 ml vol of Streptococcus pneumoniae ATCC 6303 suspended in Todd-Hewitt broth or Pseudomonas aeruginosa ATCC 27316 suspended in saline was applied to the nares of anesthetized mice (Collins, M.S. and J.H. Dorsey. 1985. Comparative in vivo activity between intravenous immune globulin prepared by reduction and alkylation or by low pH. J. Infect. Dis 151:1171-1173).

Burn Wound Sepsis. Mice were anesthetized and given a 10% body surface area burn on the back with a gas flame. A 0.5 ml vol of cells suspended in sterile saline was injected by the subcutaneous route into the burn wound. Uninfected trauma control mice received 0.5 ml saline to maintain fluid balance (Collins, M.S. and R.E. Roby, 1983. Anti-Pseudomonas aeruginosa activity of an intravenous human IgG preparation in burned mice 23:530-534).

EP 0 345 543 B1

# Table 4

## Immunoglobulin prophylaxis of peritoneal infections

| Challenge strain | Protein mg/kg | Cumulative mortality, No. dead/total Plasma-derived protein treatment | | |
| --- | --- | --- | --- | --- |
| | | Albumin | IgG | IgM |
| Streptococcus agalactiae 1b | 90 | 8/10 | 3/10 | 5/10 |
| S. agalactiae 1c | 90 | 9/10 | 1/10 | 9/10 |
| Klebsiella pneumoniae ATCC 8047 | 180 | 10/10 | 9/10 | 0/10 |
| K. pneumoniae ATCC 8047 | 180 | 10/10 | 9/10 | 0/10 |
| Total (% dead) | | 37/40 (92.5) | 22/40 (55) | 14/40 (35) |

The infections were lethal in mice; overall, 92.5% of mice treated with human serum albumin died. IgG was protective against two strains of S. agalactiae (group B Streptococcus); however, no IgG protection was evident in mice challenged with two strains of K. pneumoniae. In contrast, IgM conferred little protection to mice challenged with S. agalactiae, but IgM was highly protective in mice challenged with the Gram-negative bacillus, K. pneumoniae.

Table 5

Immunoglobulin prophylaxis of mucin-enhanced peritoneal infections

| Challenge strain | Protein mg/kg | Cumulative mortality, No. dead/total Plasma-derived protein treatment | | |
|---|---|---|---|---|
| | | Albumin | IgG | IgM |
| Escherichia coli 06 | 250 | 17/20 | 9/10 | 1/20 |
| E. coli 07:K1 | 180 | 9/10 | 8/10 | 1/10 |
| E. coli 050:K1[a] | 180 | 9/10 | 10/10 | 0/10 |
| E. coli 016:K1[a] | 180 | 9/10 | 10/10 | 0/10 |
| Serratia marcescens 06:H3 | 180 | 8/10 | 6/10 | 1/10 |
| Total (% dead) | | 52/60 (86.7) | 43/50 (86) | 3/60 (5) |

a  IgM was opsonic in the phagocytic assay (Table 2).

The infections were lethal; overall, 86.7% of albumin-treated control mice died. IgG was not protective, for 86% of treated mice died. In contrast, the overall mortality of mice treated with IgM was only 5%.

Table 6

Immunoglobulin prophylaxis of pneumonia

| | | Cumulative mortality, No. dead/total | | |
| | | Plasma-derived protein treatment | | |
| Challenge strain | Protein mg/kg | Albumin | IgG | IgM |
|---|---|---|---|---|
| S. pneumoniae ATCC 6303 serotype 3 | 40 | – | 2/20[a] | 9/20 |
| | 360 | 13/20 | 2/20[a] | 10/20 |
| P. aeruginosa ATCC 27316 | 33 | – | 12/20 | 3/10[b] |
| | 100 | – | 8/20[a] | 2/10[b] |
| | 300 | 18/20 | 8/20[a] | 1/10[b] |

a  Protection P < .05 by chi-square test vs albumin control
b  Protection P < .01 by chi-square test vs albumin control

Against S. pneumoniae IgG at 40 and 360 mg/kg was protective (P < .05). Similarly, IgG at 100 and 300 mg/kg was protective against pneumonia induced by P. aeruginosa. In contrast, IgM was not protective in mice challenged by the intranasal route with S. pneumoniae; however, IgM at each dosage level was highly protective (P < .01) against P. aeruginosa pneumonia.

11

Table 7

Immunoglobulin prophylaxis against burn wound sepsis

| Challenge strain | Protein mg/kg | Cumulative mortality, No. dead/total Plasma-derived protein treatment | | |
|---|---|---|---|---|
| | | Albumin | IgG | IgM |
| P. aeruginosa ATCC 27316 | 180 | 7/10 | 1/10 | 0/10 |
| P. aeruginosa SK 76375 | 180 | 10/10 | 8/10 | 1/10 |
| P. aeruginosa ATCC 27315 | 10 | 9/10 | 6/10 | 0/20 |
| Proteus mirabilis 4550 | 250 | 10/10 | 10/10 | 0/10 |
| Total % dead | | 36/40 (90) | 25/40 (62.5) | 1/50 (2) |

dIgG was protective against one strain of P. aeruginosa (ATCC 27316); however, little or no protection was seen against the other three challenge strains including one strain of Proteus mirabilis. In contrast, IgM was highly protective against each of the four challenge strains. Overall, only 2% of IgM-treated mice died of burn wound sepsis.

## Example 7

Protective activity of plasma derived immunoglobulin concentrates containing a high percentage (≥ 79%) of IgM versus the activity of a concentrate containing low (12%) percentage of IgM

The anti-infective activity of three lots of plasma IgM (Pd-IgM) concentrate was compared with the activity of a commercially available concentrate containing 12% IgM, 16% IgA and 72% IgG (IgGMA) (Pentaglobin[R], Biotest Pharma GmbH, Frankfurt/Main). The experimental methods employed are cited in example 3.

Table 8

| Immunoglobulin prophylaxis of Serratia marcescens mucin-enhanced peritoneal infection | | |
|---|---|---|
| Immunoglobulin mg/kg | No. mice dead/total (% dead) Treatment-(% IgM/Total Ig) | |
| | IgGMA-(12%) | Pd-IgM-(79%) |
| 0 | 10/10 (100) | 10/10 (100) |
| 10 | 10/10 (100) | 8/10 ( 80) |
| 30 | 10/10 (100) | 5/10 ( 50) |
| 90 | 9/10 ( 90) | 6/10 ( 60) |

The infection was lethal; 100% of control mice died. Overall 1 of 30 IgGMA-treated mice lived versus 11 of 30 Pd-IgM-treated mice ($X^2 = 5.21$, P = .022).

Table 9

| Immunoglobulin prophylaxis of Pseudomonas aeruginosa burn wound sepsis. | | |
|---|---|---|
| Immunoglobulin mg/kg | No. mice dead/total (% dead) Treatment-(% IgM/Total Ig) | |
| | IgGMA-(12%) | Pd-IgM-(86%) |
| 0 | 8/10 ( 80) | 8/10 ( 80) |
| 1.5 | NT[a] | 3/10 ( 30) |
| 4.4 | 10/10 (100) | 0/10 ( 0) |
| 13.3 | 10/10 (100) | 0/10 ( 0) |
| 40.0 | 5/10 ( 50) | 0/10 ( 0) |
| [a] IgGMA was not tested at this dosage | | |

Pseudomonas burn wound sepsis was lethal; 80% of control mice died. The mean protective dose of IgGMA exceeded 40 mg Ig/kg, whereas the mean protective dose of Pd-IgM was less than 1.5 mg Ig/kg. These results indicate that Pd-IgM was at least 27-fold more potent than IgGMA in this model of infection.

Table 10

| Immunoglobulin prophylaxis of polymicrobic Staphylococcus aureus - Pseudomonas aeruginosa burn wound sepsis. | | |
|---|---|---|
| Immunoglobulin mg/kg | No. mice dead/total (% dead) Treatment-(% IgM/Total Ig) | |
| | IgGMA-(12%) | Pd-IgM-(83%) |
| 0 | 8/10 ( 80) | 8/10 ( 80) |
| 8 | 7/10 ( 70) | 0/10 ( 0) |
| 24 | 7/10 ( 70) | 1/10 ( 10) |
| 73 | 3/10 ( 30) | 0/10 ( 0) |

Polymicrobic burn wound sepsis was induced by injection of 0.5 ml saline containing 130 cells of S. aureus ATCC 14154 and 58 cells of P. aeruginosa ATCC27316 into the burn wound. The infection was lethal; 80% of control mice died. The mean protective dose of IgGMA was 45.1 mg/kg whereas the mean protective dose of Pd-IgM was less than 8 mg/kg. This indicates that Pd-IgM was at least 6-fold more potent than IgGMA in this model of infection. Furthermore, 13 of 30 IgGMA-treated mice survived versus 29 of 30 Pd-treated mice ($X^2$-10.16, P < .01).

It is intended that the invention disclosed herein should be limited only by the following claims.

**Claims**

**Claims for the following Contracting States : AT, BE, CH, DE, FR, GB, IT, LI, LU, NL, SE**

1. An intravenously administrable lyophilized IgM concentrate substantially free of isoagglutinins, derived from a plasma source.

2. The concentrate of Claim 1, wherein the concentration of IgM is at least 33 % by weight of total antibodies and the isoagglutinin titer is less than about 1:4.

3. The concentrate of any of Claims 1 and 2, wherein the concentration of IgM is at least 50 % by weight.

4. The concentrate of any of Claims 1 to 3 substantially free of anti-A antibodies.

5. The concentrate of any of Claims 1 to 4 substantially free of anti-B antibodies.

6. The concentrate of any of Claims 1 to 5, wherein reconstitution in aqueous solution results in a pH less than about 7.0.

7. Use of a concentrate according to any of Claims 1 to 6 for the manufacture of a composition in the treatment of a mammal to provide a therapeutic or prophylactic effect against gram negative bacteria

8. Use according to Claim 7, wherein the gram negative bacteria is selected from the group Escherichia coli, Serratia marcescens, Pseudomonas aeruginosa, Klebsiella pneumoniae and Proteus mirabilis

**Claims for the following Contracting States : ES, GR**

1. A method of preparing an IgM concentrate, comprising the combination of the steps of (a) concentrating the IgM to at least 33 % by weight of total antibodies, and (b) contacting the antibodies with an adsorbent under conditions sufficient to reduce anti-A or anti-B isoagglutinins to a titer of less than about 1:4.

2. The method of claim 1 wherein the adsorbent adsorbs anti-A antibodies.

3. The method of claim 1 wherein the adsorbent adsorbs anti-B antibodies.

4. The method of claim 1 wherein the adsorbent is either fixed red blood cells or fixed red blood cells stroma.

**Patentansprüche**

**Patentansprüche für folgende Vertragsstaaten : AT, BE, CH, DE, FR, GB, IT, LI, LU, NL, SE**

1. Intravenös verabreichbares lyophilisiertes IgM-Konzentrat, das im wesentlichen frei von Isoagglutininen ist, und welches aus einer Plasma-Quelle abgeleitet ist.

2. Konzentrat gemäß Anspruch 1, worin die Konzentration an IgM mindestens 33 Gew.% der Gesamt-Antikörper und der Isoagglutinin-Titer weniger als ca. 1:4 betragen.

3. Konzentrat gemäß jedem Anspruch 1 und 2, worin die Konzentration an IgM mindestens 50 Gew.% beträgt.

**4.** Konzentrat gemäß jedem Anspruch 1 bis 3, welches im wesentlichen frei von anti-A-Antikörpern ist.

**5.** Konzentrat gemäß jedem Anspruch 1 bis 4, welches im wesentlichen frei von anti-B-Antikörpern ist.

**6.** Konzentrat gemäß jedem Anspruch 1 bis 5, worin eine Rekonstitution in wässriger Lösung einen pH von weniger als ca. 7,0 ergibt

**7.** Verwendung des Konzentrats gemäß jedem Anspruch 1 bis 6 zur Herstellung einer Zusammensetzung zur Behandlung eines Säugetiers zur Bereitstellung eines therapeutischen oder prophylaktischen Effekts gegen gramnegative Bakterien.

**8.** Verwendung gemäß Anspruch 7, worin die gramnegativen Bakterien aus der Gruppe Escherichia coli, Serratia marcescens, Pseudomonas aeruginosa, Klebsiella pneumoniae und Proteus mirabilis ausgewählt sind.

**Patentansprüche für folgende Vertragsstaaten : ES, GR**

**1.** Verfahren zur Herstellung eines IgM-Konzentrats, wobei man die Stufen (a), in der man das IgM auf mindestens 33 Gew.% der Gesamt-Antikörper Konzentriert, und (b) kombiniert, in welcher man die Antikörper mit einem Adsorbens unter Bedingungen in Kontakt bringt, die ausreichen, um anti-A- oder anti-B-Isoagglutinine auf einen Titer von weniger als ca. 1:4 herabzusetzen.

**2.** Verfahren gemaß Anspruch 1, worin das Adsorbens anti-A-Antikörper adsorbiert.

**3.** Verfahren gemäß Anspruch 1, worin das Adsorbens anti-B-Antikörper adsorbiert.

**4.** Verfahren gemäß Anspruch 1, worin das Adsorbens entweder durch fixierte rote Blutzellen oder durch fixiertes Stroma roter Blutzellen dargestellt ist.

**Revendications**
**Revendications pour les Etats contractants suivants : AT, BE, CH, DE, FR, GB, IT, LI, LU, NL, SE**

**1.** Concentré d'IgM lyophilisé dérivé d'une source plasmatique, essentiellement exempt d'isoagglutinines, pouvant être administré par voie intraveineuse.

**2.** Concentré selon la revendication 1, dans lequel la concentration d'IgM est d'au moins 33 % en masse des anticorps totaux, et le titre d'isoagglutinines est inférieur à environ 1:4.

**3.** Concentré selon l'une quelconque des revendications 1 et 2, dans lequel la concentration d'IgM est d'au moins 50 % en masse.

**4.** Concentré selon l'une quelconque des revendications 1 à 3, essentiellement exempt d'anticorps anti-A.

**5.** Concentré selon l'une quelconque des revendications 1 à 4, essentiellement exempt d'anticorps anti-B.

**6.** Concentré selon l'une quelconque des revendications 1 à 5, pour lequel la reconstitution en solution aqueuse donne un pH inférieur à environ 7,0.

**7.** Utilisation d'un concentré selon l'une quelconque des revendications 1 à 6 pour la préparation d'une composition de traitement d'un mammifère procurant un effet thérapeutique ou prophylactique contre des bactéries gram négatives.

**8.** Utilisation selon la revendication 7, dans laquelle les bactéries gram négatives sont choisies dans le groupe constitué par *Escherichia coli, Serratia marcescens, Pseudmonas aeruginosa, Klebsiella pneumoniae et Proteus mirabilis.*

**Revendications pour les Etats contractants suivants : ES, GR**

1.  Méthode de préparation d'un concentré d'IgM, comprenant la combinaison des étapes selon lesquelles (a) on concentre l'IgM à au moins 33 % en masse des anticorps totaux, et (b) on met les anticorps en contact avec un adsorbant dans des conditions suffisantes pour réduire le titre d'isoagglutinines anti-A ou anti-B à une valeur inférieure à environ 1:4.

2.  Méthode selon la revendication 1, dans laquelle l'adsorbant adsorbe les anticorps anti-A.

3.  Méthode selon la revendication 1, dans laquelle l'adsorbant adsorbe les anticorps anti-B.

4.  Méthode selon la revendication 1, dans laquelle l'adsorbant est constitué soit par des globules rouges fixés, soit par des stromas de globules rouges fixés.